# EUROPEAN PATENT APPLICATION

(11) **EP 3 321 211 A1**
(43) Date of publication of application: **16.05.2018**
(21) Application number: 17200849.2
(22) Date of filing: 09.11.2017
(51) Int. Cl.: B65D 83/40, A61M 15/00

(54) **INHALER DEVICE COMPRISING A FIXABLE LID ASSEMBLY**

(30) Priority: 09.11.2016 TR 201616027; 22.12.2016 TR 201619235; 30.12.2016 TR 201620257; 08.11.2017 TR 201717514
(71) Applicant: Arven Ilac Sanayi Ve Ticaret A.S., Istanbul 34460 (TR)
(72) Inventor: TOKSÖZ, Ahmet, 34460 Istanbul (TR); TOKSÖZ, Zafer, 34460 Istanbul (TR)
(74) Representative: Sevinç, Erkan

(57) **Abstract**

The present invention relates to an inhaler device (1) comprising a mouthpiece (40) positioned on an outer body (30), a lid (10) that can be brought to open and closed positions on said mouthpiece (40) and a hinge (20) joining said lid (10) and the outer body (30), characterized by comprising at least one locking means (100) that enables the lid (10) to be fixed in the open and/or closed position.

## Description

### Technical Field

The present invention relates to an inhaler device comprising a fixable lid that enables the user to inhale in dry powder form the medicaments used for the treatment of diseases such as asthma, bronchitis and COPD (Chronic Obstructive Pulmonary Disease), etc.

### Prior Art

Despite the advances in diagnosis and treatment in recent years, diseases such as asthma, bronchitis and COPD (Chronic Obstructive Pulmonary Disease) decrease the quality of life of humans. The administration of medicaments by means of inhalers is recommended for optimizing the treatment of these types of diseases. The treatment by means of inhalers is the most preferred treatment type and is expected to be the first option in the future. The most important advantage of using medicaments by means of inhalation is to provide a more efficient treatment by using less medicaments, to administer medicaments in a higher concentration the respiratory tract and in particular to decrease the systemic side effects of medicaments. Despite the presence of very efficient treatments for respiratory tract diseases, the most significant reasons of failing to properly control patients are stated as noncompliance with the treatments recommended by the physicians and incoordination due to the improper use of inhalers.

In recent years, various inhalation devices have been developed for administering inhalation medicaments. These devices are basically categorized into two types: metered-dose inhalers and dry powder inhalers. These types of devices structurally comprise basic components such as mechanism, counter, body, lid, lock, etc. In addition, powder inhalation medicaments are preserved in carriers such as blister, capsule, etc. Blisters are composed of two basic components, namely the main layer with cavities for carrying the medicament and the strippable lid layer.

The type of dry powder inhaler devices with blisters comprises a mechanism that moves the blister forward and provides the sequential use of cavities in the blister. The patient uses an arm or a trigger to move said mechanism. This type of devices has main sections such as advancement mechanism, counter and lock and mechanism that form the same. Said sections and mechanisms are positioned and protected in an outer body.

In the state of the art inhaler devices, problems arise in the lid of the device while using the medicament. It is aimed to protect the device and the mouthpiece used by the patient to inhale the medicament by means of a lid. The lid prevents the mouthpiece from being in contact with the outer environment, thus providing the use of a more sterile device. However, said lids cannot be kept fixed when in the open position in the state of the art inhaler devices. This situation causes some inconveniences. When the patient opens the lid of the device in order to inhale the powder medicament from the inhaler device, the lid moves during the use of the medicament and hits the face of the patient. In particular, due to the rapid and abrupt movements of the patient in unexpected cases where the patient must take the medicament urgently, the moving lid may prevent the patient from taking the medicament and the lid may be broken while the patient tries to control the same. The inhaler devices having patient compliance is considered as the first priority in mechanisms used for the administration of medicaments. Problems encountered in the state of the art cause dysfunctional use.

In the relevant technical field, an inhaler device that can be operated with the desired precision and that provides comfortable and safe use to the patient is required in order to eliminate said problems in the inhaler devices.

### Aims and Brief Description of the Invention

The present invention relates to an improved inhaler device used in dry powder inhalation operation, that eliminates all the above-mentioned problems and that provides advantages to the relevant technical field.

With respect to the state of the art, the principal aim of the present invention is to provide an inhaler device comprising a locking mechanism fixing the lid of the device that is open while the user takes the powder medicament and that meanwhile falls towards the face of the user.

Another aim of the present invention is to provide an inhaler device that enables the lid of the device used in an abrupt manner by the user in case of emergency to be kept fixed and thus enables the user to completely inhale the powder medicament from the device.

Yet another aim of the present invention is to allow a more sterile use of the device by enabling the lid to be locked after being closed following the use of the inhaler device by the user.

Yet another aim of the present invention is to ensure the use of the inhaler device with a lid assembly enabling the patient compliance to be increased.

The present invention is an inhaler device comprising a mouthpiece positioned on an outer body, a lid that can be brought to open and closed positions on said mouthpiece and a hinge joining said lid and the outer body, characterized by comprising at least one locking means that enables the lid to be fixed in the open and/or closed position.

The present invention is an inhaler device characterized in that said locking means comprises at least one pin and at least one upper guide corresponding to the said pin.

The present invention relates to an inhaler device characterized in that said locking means comprises at least one pin positioned on at least one surface of the hinge for fixing the lid in the open position when the lid is brought to the open position by means of a force exerted on the lid, and at least one upper guide that is disposed on the hinge housing of the outer body so as to be aligned with said pin and that enables the lid to be fixed in the open position by being locked as the pin pivots and pushes the upper guide as a result of the rotational movement.

The present invention relates to an inhaler device characterized in that said locking means comprises at least one pin positioned on at least one surface of the hinge for fixing the lid in the closed position by means of a force exerted on the lid, and at least one lower guide that is disposed on the hinge housing of the outer body so as to be aligned with said pin and that enables the lid to be fixed in the closed position by being locked as the pin pivots and pushes the lower guide as a result of the rotational movement.

The present invention relates to an inhaler device characterized by comprising at least one pin positioned as a single piece with the hinge.

The present invention relates to an inhaler device characterized by comprising at least one pin positioned as a single piece with the hinge on both surfaces of the hinge, namely the front surface and the rear surface.

The present invention relates to an inhaler device characterized by comprising two identical pins positioned with a predetermined distance therebetween both on the front surface and the rear surface of the hinge in the same linear alignment.

The present invention relates to an inhaler device characterized by comprising at least one upper guide and at least one lower guide that are produced from a flexible material and that are positioned on the hinge housing.

### Brief Description of Figures

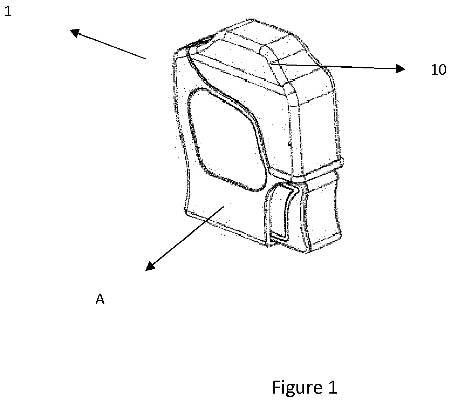

Figure 1 is the front perspective view of the inhaler device of the present invention in the closed position.

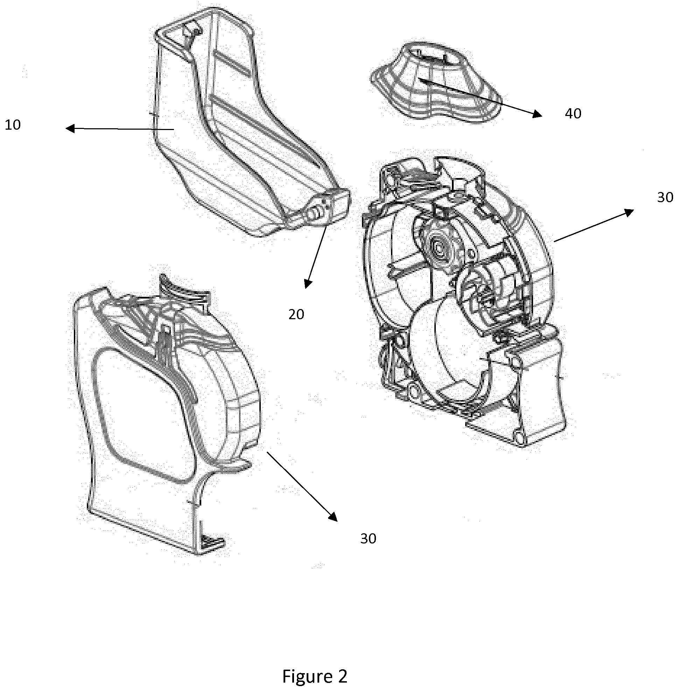

Figure 2 is the exploded view of the inhaler device of the present invention.

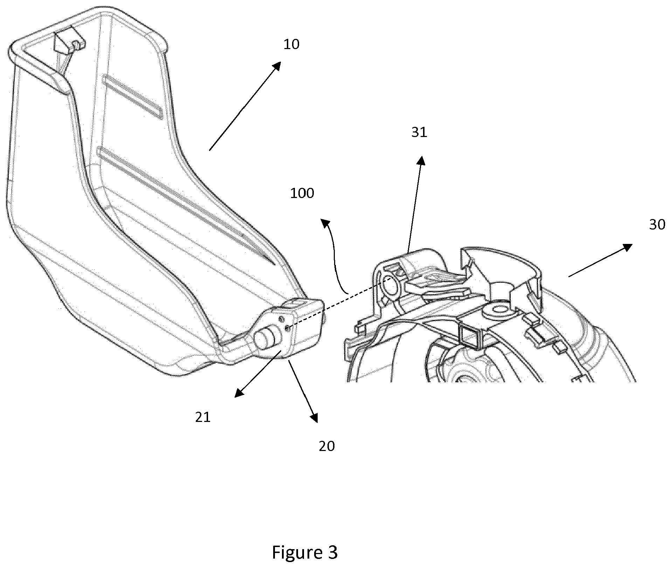

Figure 3 is the cross-sectional view of the lid and the outer body of the inhaler device of the present invention.

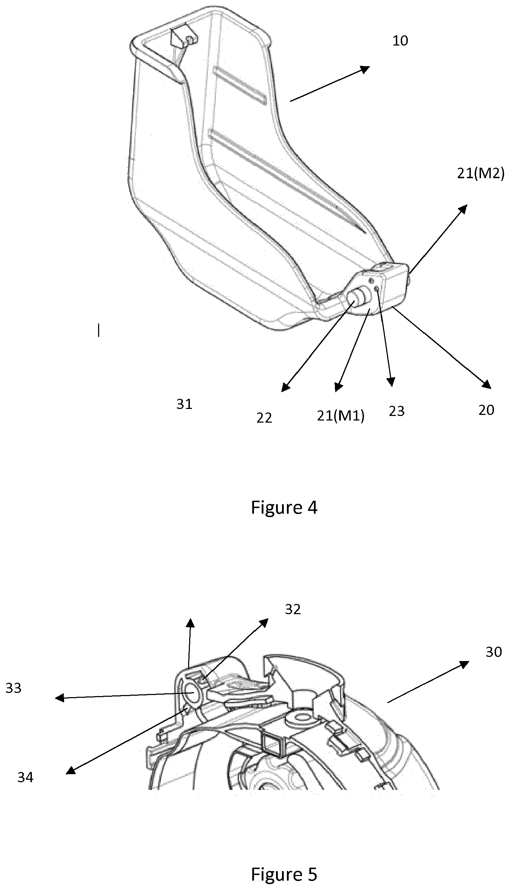

Figure 4 is the front perspective view of the lid of the inhaler device of the present invention.
Figure 5 is the front perspective cross-sectional view of the outer body of the inhaler device of the present invention.

### Reference Numbers in Figures

1. Inhaler device
   10. Lid
   20. Hinge
      21. Hinge surface
      22. Hinge connection element
      23. Pin
   30. Outer body
      31. Hinge housing
      32. Upper guide
      33. Hinge connection element housing
      34. Lower guide
   40. Mouthpiece
100: Locking means
A: Main body: Body formed by joining two identical outer bodies which are positioned to face each other.
M1, M2: Hinge comprises a hinge front surface (M1) and a hinge rear surface (M2).

### Detailed Description of the Invention

The inhaler device (1) of the present invention is explicated in an exemplary manner referring to the attached figures only so as to clarify without introducing any limiting effects.

The inhaler device (1) of the present invention comprises a main body (A) that is formed by joining two identical outer bodies (30) and that has a blister wherein the powder medicament is disposed and a movement mechanism that enables the powder medicament in the blister to be used; a mouthpiece (40) that is positioned at the mouth portion of the outer body (30) and that enables the user to inhale the powder medicament, and a lid (10) that is opened by pivoting when the inhaler device (1) is to be used and that is closed by pivoting when the inhaler device (1) is not used so as to prevent access to the mouthpiece (40) and to provide the safety of the inhaler device (1).

In the inhaler device (1) of the present invention, the connection between the lid (10) and the outer body (30) is provided by a hinge (20). Said hinge (20) enables the lid (10) to be shifted between the open and closed positions on the mouthpiece (40) disposed at the mouth portion of the outer body (30).

The lid (10) is shifted between the open and closed positions on the mouthpiece (40) by means of the pivot movement of the hinge (20) positioned as integrated with the lid (10) at the end portion of the lid (10) in the hinge housing (31) corresponding to said hinge (20) and positioned on the outer body (30).

The hinge (20) comprises at least one hinge surface (21); at least one hinge connection element (22) that is positioned on said hinge surface (21) and that enables the lid (10) to shift between the open and closed positions on the mouthpiece (40), and at least one pin (23) for keeping the lid (10) fixed in the open or closed position.

In the principal embodiment of the present invention, the hinge connection element (22) and the pin (23) that are positioned on the hinge (20) and are single piece with the hinge (20).

In a preferred embodiment of the present invention, at least one hinge connection element (22) and at least one pin (23) are positioned on each of both surfaces (M1, M2) of the hinge (20) and are single piece with the both front surface (M1) and the rear surface (M2).

The hinge housing (31) comprises a hinge connection element housing (33) wherein the hinge connection element (22) is fitted so as to enable the lid (10) to move between the open and closed positions on the mouthpiece (40); an upper guide (32) to which the pin (23) is fixed by pushing the same with a pivot movement by means of a force exerted on the lid (10) so as to keep the lid (10) in the open position, and a lower guide (34) to which the pin (23) is fixed by pushing the same with a pivot movement by means of a force exerted on the lid (10) so as to keep the lid (10) in the closed position.

In a preferred embodiment of the present invention, the hinge housing (31) constitutes a single piece with the upper guide (32), the hinge connection element housing (33) and the lower guide (34) that are positioned on the hinge housing (31).

A locking means (100) enables the lid (10) to be fixed in the open or closed position on the mouthpiece (40). Said locking means (100) comprises at least one pin (23) and at least one guide (32 or 34) corresponding to said pin (23).

In an alternative embodiment of the present invention, the locking means (100) comprises a pin (23) and an upper guide (32) corresponding to said pin (23) and/or a pin (23) and a lower guide (34) corresponding to said pin (23) and/or a pin (23) and a lower guide (34) and an upper guide (32) both corresponding to said pin (23) and/or a pin (23) and an upper guide (32) corresponding to said pin (23) and a second pin (23) and a lower guide (34) corresponding to said second pin (23).

In a preferred embodiment of the present invention during the assembly of the inhaler device (1), the lid (10) is disposed between the two identical outer bodies (30) such that the hinge connection element (22) on each surface (M1, M2) of the hinge (20) is positioned in the hinge connection element housing (33) on the hinge housing (31) on each outer body (30).

The user opens the lid (10) of the inhaler device (1) so as to take the powder medicament. The hinge connection element (22) and the hinge connection element (22) disposed respectively on the identical front surface (M1) and rear surface (M2) of the hinge (20) are pivoted in the hinge connection element housing (33) positioned on the hinge housing (31) on each outer body (30) so as to make a rotational movement. With this movement, the lid (10) is brought to the open position. During the administration of the dry powder medicament by means of the mouthpiece (40), the movement of the lid (10) is prevented by means of the locking means (100) as the pin (23) on the front surface (M1) of the hinge (20) pushes the upper guide (32) positioned in the hinge housing (31) of the outer body (30) with a pivot movement so as to be locked to the upper guide (32) and the pin (23) on the rear surface (M2) of the hinge (20) pushes the upper guide (32) positioned in the hinge housing (31) of another outer body (30) with a pivot movement so as to be locked to the upper guide (32). This locking is realized with a clicking sound and the lid (10) is fixed in the open position.

Similarly, the lid (10) is moved in a direction opposite to the opening direction in order to cover the mouthpiece (40) after the administration of the dry powder medicament is completed. The lid (10) is locked in the closed position by means of the locking means (100) as the pin (23) on the front surface (M1) of the hinge (20) pushes the lower guide (34) positioned in the hinge housing (31) of the outer body (30) with a pivot movement so as to be locked to the lower guide (34) and the pin (23) on the rear surface (M2) pushes the lower guide (34) positioned in the hinge housing (31) of another outer body (30) with a pivot movement so as to be locked to the lower guide (34). This locking is realized with a clicking sound and the lid (10) is fixed in the closed position.

In a preferred embodiment of the present invention, the locking means (100) enabling the lid (10) to be fixed in the open and closed positions comprises two identical pins (23) with a predetermined distance therebetween both on the front and rear surfaces (M1, M2) of the hinge (20) in the same linear alignment. As one of the pins (23) pivots and pushes the upper guide (32) so as to be fixed to the upper guide (32) in order to fix the lid (10) in the open position, the locking is realized, and as the other pin (23) pivots and pushes the lower guide (34) so as to be fixed to the lower guide (34) in order to fix the lid (10) in the closed position, the locking is realized.

Existing components are polymer-based, and produced and shaped by injection molding method.

Consequently, by means of the embodiment of the present invention, an inhaler device (1) is realized, providing a high-precision, smooth operation and comprising a lid (10) that can be fixed in the open and closed positions during and after the use of said device (1).

## Claims

1. An inhaler device (1) comprising a mouthpiece (40) positioned on an outer body (30), a lid (10) that can be brought to open and closed positions on said mouthpiece (40) and a hinge (20) joining said lid (10) and the outer body (30), **characterized by** comprising at least one locking means (100) that enables the lid (10) to be fixed in the open and/or closed position.

2. An inhaler device (1) as in Claim 1, **characterized in that** said locking means (100) comprises at least one pin (23) and at least one upper guide (32) corresponding to the said pin (23).

3. An inhaler device (1) as in any one of the above claims, **characterized in that** said locking means (100) comprises at least one pin (23) positioned on at least one surface (M1, M2) of the hinge (20) for fixing the lid (10) in the open position when the lid (10) is brought to the open position by means of a force exerted on the lid (10), and at least one upper guide (32) that is disposed on the hinge housing (31) of the outer body (30) so as to be aligned with said pin (23) and that enables the lid (10) to be fixed in the open position by being locked as the pin (23) pivots and pushes the upper guide (32) as a result of the rotational movement.

4. An inhaler device (1) as in any one of the above claims, **characterized in that** said locking means (100) comprises at least one pin (23) positioned on at least one surface (M1, M2) of the hinge (20) for fixing the lid (10) in the closed position by means of a force exerted on the lid (10), and at least one lower guide (34) that is disposed on the hinge housing (31) of the outer body (30) so as to be aligned with said pin (23) and that enables the lid (10) to be fixed in the closed position by being locked as the pin (23) pivots and pushes the lower guide (34) as a result of the rotational movement.

5. An inhaler device (1) as in any one of the above claims, **characterized by** comprising at least one pin (23) positioned as a single piece with the hinge (20).

6. An inhaler device (1) as in any one of the above claims, **characterized by** comprising at least one pin (23) positioned as a single piece with the hinge (20) on both surfaces (M1, M2) of the hinge (20), namely the front surface (M1) and the rear surface (M2).

7. An inhaler device (1) as in any one of the above claims, **characterized by** comprising two identical pins (23) positioned with a predetermined distance therebetween both on the front surface (M1) and the rear surface (M2) of the hinge (20) in the same linear alignment.

8. An inhaler device (1) as in any one of the above claims, **characterized by** comprising at least one upper guide (32) and at least one lower guide (34) that are produced from a flexible material and that are positioned on the hinge housing (31).
